**Europäisches Patentamt**

**European Patent Office**

**Office européen des brevets**

(19)

(11) Numéro de publication : **0 491 611 A1**

## (12) DEMANDE DE BREVET EUROPEEN

(21) Numéro de dépôt : **91403404.6**

(22) Date de dépôt : **16.12.91**

(51) Int. Cl.⁵ : **C01B 33/12, B01J 8/24**

(30) Priorité : **17.12.90 FR 9015765**

(43) Date de publication de la demande :
**24.06.92 Bulletin 92/26**

(84) Etats contractants désignés :
**AT BE CH DE DK ES FR GB GR IT LI LU NL SE**

(71) Demandeur : **ELECTRICITE DE FRANCE**
**Service National**
**2, rue Louis Murat**
**F-75008 Paris (FR)**

(72) Inventeur : **Baudequin, François**
**27, rue du Bois Meslé**
**F-95600 Eaubonne (FR)**

(74) Mandataire : **Martin, Jean-Jacques et al**
**Cabinet REGIMBEAU 26, Avenue Kléber**
**F-75116 Paris (FR)**

(54) **Procédé de traitement de fumées de silice en vue de leur blanchiment, et dispositif de mise en oeuvre du procédé.**

(57) L'invention concerne un procédé de traitement de fumées de silice en vue de leur blanchiment, et un dispositif de mise en oeuvre de ce procédé.

Conformément à l'invention, la poudre noire à traiter (105) est introduite en partie basse d'un lit dense (113) fluidisé et chauffé, ledit lit présentant une hauteur efficace (h) permettant d'oxyder en gaz carbonique le carbone des particules de silice lorsque celles-ci traversent le lit dense fluidisé (113) en étant entraînées par l'air de fluidisation ; la poudre alors blanchie (131) est récupérée par filtration des gaz issus dudit lit dense fluidisé.

Application notamment au domaine des cosmétiques pour des produits utilisant des poudres de silice.

L'invention concerne le traitement de fumées de silice, et en particulier des fumées de silice s'échappant de fours électriques d'élaboration de silicium.

Les fours électriques utilisés pour la fabrication de silicium sont des fours à arc dotés d'électrodes en graphite. Du fait des températures très élevées et de l'aérothermie interne de ces fours, une certaine proportion de silicium s'échappe dans les fumées et s'oxyde en silice.

Ces fumées de silice, de formule $SiO_2$, présentent une granulométrie très fine (de l'ordre de quelques µm) et sont toujours de couleur noire. Cette couleur est essentiellement due à la captation par les particules de silice du carbone provenant de l'usure des électrodes en graphite.

La couleur noire de ces fumées de silice les rend impropre à une utilisation valorisante, notamment dans le domaine de l'industrie des **cosmétiques,** bien que l'existence d'une granulométrie très fine pousse les spécialistes à rechercher une application qui exploiterait les avantages inhérents à la possibilité de disposer directement d'une poudre de silice de très faible granulométrie.

Il convient alors de procéder au blanchiment des fumées de silice, afin de pouvoir valoriser ces fumées.

Cependant, l'homme de l'art se heurte à de grandes difficultés s'il veut utiliser les procédés d'incinération traditionnels qui sont à sa disposition, tels que les procédés utilisant un four tournant ou un calcinateur rapide (dénommé "calcinateur flash" par les spécialistes du domaine).

En effet, si l'on utilise un four tournant de conception classique, qui se présente sous la forme d'un cylindre tournant dont l'axe est légèrement incliné par rapport à l'horizontale et d'une flamme disposée à l'entrée ou la sortie du cylindre, on constate que le produit injecté dans le cylindre pour y être retourné colle et se réagglomère en amas de plus grande taille, perdant ainsi sa finesse.

Ceci s'explique par le fait que les deux paramètres temps de contact et température ne permettent pas d'avoir à la fois un blanchiment de la poudre et un maintien de la granulométrie très fine de cette poudre : en effet, le temps de contact étant de facto relativement court, soit la température est choisie à une valeur peu élevée pour éviter le collage du produit, mais alors on parvient à une poudre qui reste de couleur plus ou moins grise, soit la température est poussée pour parvenir à un blanchiment satisfaisant, mais alors on dépasse la température de frittage de la poudre et on obtient un produit se présentant sous la forme de blocs agglomérés.

On pourrait certes imaginer une chaîne de traitement supplémentaire pour ramener ces blocs agglomérés à des particules de granulométrie plus acceptable, mais le processus deviendrait encore plus onéreux, et entraînerait de toute façon une altération de leur géométrie (les particules écrasées seraient en effet très rugueuses, et cette absence de sphéricité les rendrait impropre à une utilisation dans laquelle on recherche une grande onctuosité pour un produit en poudre, par exemple dans l'industrie des cosmétiques pour la composition des fonds de teint et des "poudre de riz").

Par ailleurs, si l'on utilise un calcinateur flash de conception classique, qui se présente sous la forme d'un cylindre vertical étroit de grande hauteur et à la base duquel on injecte de l'air chaud à vitesse élevée, chaque grain de poudre sustenté isolément sous la forme d'un "lit entraîné" est soumis à une température qui peut être maintenue inférieure à la température de frittage (pour éviter un collage du produit), mais le temps de mise en contact de la poudre avec l'air à haute température est insuffisant (inférieur à la seconde), de sorte que la poudre récupérée en aval du calcinateur est là encore de couleur grise.

On pourrait certes chercher à augmenter le temps de contact en prévoyant une hauteur supérieure à la hauteur traditionnelle des calcinateurs existants, laquelle est en général de l'ordre de 12 à 15 m, mais on arriverait alors à une installation énorme car la hauteur devrait dépasser au moins une cinquantaine de mètres.

Ainsi, les procédés traditionnels ne permettent pas de réaliser un blanchiment satisfaisant des fumées de silice tout en maintenant leur granulométrie à une valeur très basse.

Il convient également de noter que l'homme de l'art écarterait a priori toute technique de fluidisation par un gaz chaud d'un lit de poudre à traiter, car la granulométrie de cette poudre est très inférieure à la limite des poudres dites fluidisables (en général 30 à 50 µm) : en effet, les grains soumis aux forces interparticulaires et aux forces de gravité ne peuvent plus dans ce cas être sustentés par un courant de fluidisation, et on ne parvient pas à éviter que la poudre s'envole.

L'invention a pour objet de résoudre ce problème, en réalisant un procédé de traitement et un dispositif de mise en oeuvre de ce procédé, qui permettent de réaliser un blanchiment des fumées de silice tout en préservant la finesse de leur granulométrie, ce qu'on ne parvenait pas à obtenir avec les procédés traditionnels indiqués plus haut.

L'invention a également pour objet de proposer un procédé et un dispositif de mise en oeuvre qui soient exploitables dans des conditions financières raisonnables, sans impliquer un dimensionnement important pour l'installation de traitement, et avec la possibilité de piloter automatiquement le processus de traitement.

L'invention a aussi pour objet de proposer un procédé et un dispositif de mise en oeuvre faisant appel à des moyens dont la structure reste simple et fiable, permettant de connecter aisément l'installation de traitement au reste de l'usine en automatisant l'alimentation en fumées de silice et la collecte du produit fini.

Il s'agit plus particulièrement d'un procédé de traitement de fumées de silice en vue de leur blanchiment, caractérisé par le fait que la poudre noire à traiter est introduite en partie basse d'un lit dense fluidisé et chauffé, ledit lit présentant une hauteur efficace prédéterminée entre le niveau d'introduction et la surface du lit permettant d'oxyder en gaz carbonique le carbone des particules de silice lorsque celles-ci traversent le lit dense fluidisé en étant entraînées par l'air de fluidisation, la poudre alors blanchie étant récupérée par filtration des gaz issus dudit lit dense fluidisé.

De préférence, le lit dense est un lit de sable, dont la hauteur efficace est choisie en fonction du diamètre moyen des particules de silice à traiter.

Avantageusement alors, le diamètre moyen des particules de sable constituant le lit dense est de l'ordre de 400 µm. En particulier, la hauteur efficace du lit dense est de l'ordre de 1,5 mètre lorsque ledit lit est fluidisé.

De préférence, le lit dense est chauffé par sa surface extérieure, par chauffage électrique. Dans le cas d'une installation devant traiter un tonnage élevé de poudre (par exemple plus de trois tonnes par heure), on pourra prévoir en variante un chauffage électrique immergé au sein du lit fluidisé, par exemple sous la forme d'un termoplongeur de type traditionnel.

Avantageusement, le lit dense est chauffé sur la totalité de sa hauteur efficace, de façon que tout l'air de fluidisation soit de l'air comburant pour oxyder le carbone présent dans les fumées de silice, la température dudit lit restant cependant inférieure à la température de frittage de la poudre à traiter.

De préférence, la température du lit dense peut être réglée entre environ 550°C et 850°C pour oxyder complètement le carbone sans modifier l'état cristallographique des fumées de silice.

Il est par ailleurs intéressant que les gaz issus du lit dense fluidisé passent par un séparateur cyclone, pour ramener les éventuelles particules de sable vers le lit dense, la filtration qui permet de récupérer la poudre blanchie étant effectuée en aval dudit séparateur cyclone.

De préférence alors, la séparation opérée par le séparateur cyclone est obtenue par le choix d'un diamètre de coupure de l'ordre de 30 µm pour ledit séparateur cyclone.

Il est également intéressant de prévoir que les gaz sortant du séparateur cyclone subissent une dilution avec de l'air frais pour abaisser leur température avant que ces gaz soient filtrés pour la récupération de la poudre blanchie. En particulier, la température des gaz est ramenée à moins de 150°C avant leur filtration.

L'invention concerne également un dispositif de mise en oeuvre du procédé de traitement précité, ce dispositif étant caractérisé par le fait qu'il comporte une enceinte fermée contenant un lit dense fluidisé par des moyens d'amenée d'air et maintenu à une température déterminée par des moyens de chauffage associés, des moyens d'introduction de la poudre noire à traiter en partie basse du lit dense fluidisé, ledit lit présentant une hauteur efficace prédéterminée entre le niveau d'introduction et la surface du lit, et ladite enceinte se prolongeant supérieurement, au-dessus de la surface du lit dense fluidisé, par une chambre de désengagement reliée à un circuit de séparation pour récupérer la poudre blanchie, ledit circuit de séparation comportant des moyens de filtration des gaz issus du lit dense fluidisé et des moyens de récupération de la poudre blanchie.

De préférence, l'enceinte fermée est métallique, et sa paroi extérieure est chauffée par des moufles chauffants électriques. En particulier, les moufles chauffants électriques s'étendent sur une hauteur au moins sensiblement égale à la hauteur efficace prédéterminée du lit dense fluidisé. Ainsi que cela a été dit plus haut, il est possible d'utiliser en variante un thermoplongeur immergé dans le lit dense fluidisé pour des installations importantes.

De préférence également, l'enceinte fermée contient un lit dense de sable dont la hauteur efficace est de l'ordre de 1,5 mètre lorsque ledit lit est fluidisé, et présente une chambre de désengagement dont la hauteur est de plusieurs mètres, en particulier au moins trois mètres. Le diamètre de l'enceinte fermée est moins critique, mais doit cependant être supérieur à une valeur minimale pour éviter tout risque de formation d'une succession de pistons de solide/gaz, ce qui nuirait à un bon échange thermique : on pourra par exemple choisir dans ce cas un diamètre de l'ordre d'un mètre.

Il est également intéressant que les moyens d'amenée d'air comportent une conduite unique d'amenée raccordée à l'enceinte fermée par le fond de celle-ci.

De préférence aussi, les moyens d'introduction de la poudre noire à traiter comportent une trémie et une vis de transport sensiblement horizontale débouchant à l'intérieur de l'enceinte fermée. Toutefois, dans le cas d'une installation importante (par exemple destinée à traiter plus de trois tonnes par heure), il sera préférable de prévoir plusieurs points d'introduction répartis sur la périphérie de l'enceinte fermée, pour que les particules soient réparties uniformément dans la section du lit dense fluidisé.

Il est par ailleurs avantageux que le circuit de séparation comporte un séparateur cyclone dont l'entrée est directement reliée à la chambre de désengagement de l'enceinte fermée, le diamètre de coupure dudit séparateur cyclone étant de préférence choisi voisin de 30 µm.

De préférence encore, les moyens de filtration sont essentiellement constitués par un filtre à manches, dont la partie inférieure aboutit à un sas rotatif sous lequel est disposé un bac constituant les moyens de récupération, et dont la partie supérieure est reliée à un ventilateur d'exhaure évacuant les gaz vers une cheminée. En particulier, le filtre à manches est en textile, et présente un diamètre de coupure au plus égal à 1 µm.

Avantageusement encore, la conduite reliant la sortie du séparateur cyclone à l'entrée du filtre à manches comporte une dérivation ouverte permettant d'admettre dans ladite conduite de l'air froid de dilution. De préférence alors, la dérivation comporte un organe de réglage tel qu'une clé, pour régler la quantité d'air de dilution arrivant dans ladite conduite.

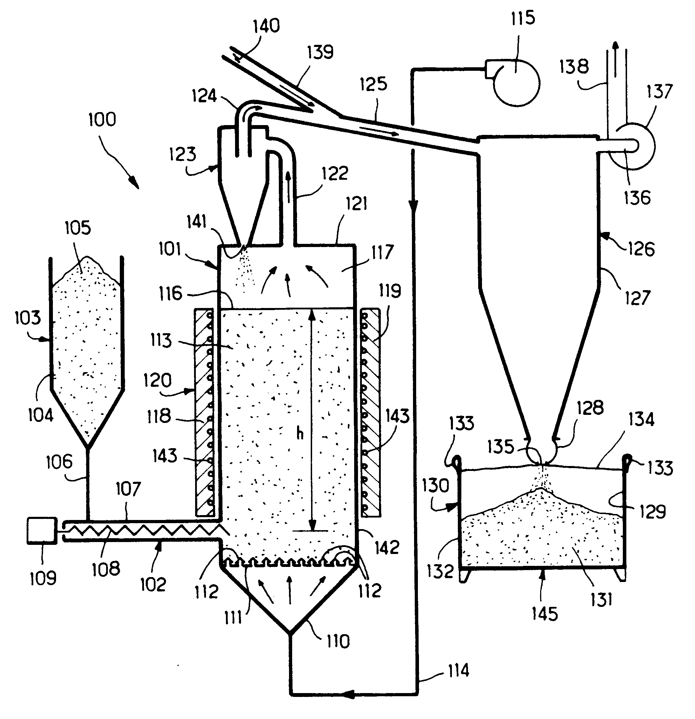

D'autres caractéristiques et avantages de la présente invention ressortiront plus clairement à la lumière de la description qui va suivre et du dessin annexé, concernant un mode de réalisation particulier, en référence à la figure unique qui illustre schématiquement une installation de traitement conforme à l'invention.

L'installation de traitement 100 illustrée sur la figure unique comporte une enceinte fermée 101 contenant un lit dense 113 fluidisé par des moyens d'amenée d'air associés 111, 112, 114, 115, et maintenu à une température déterminée par des moyens de chauffage 120 associés. L'enceinte 101 comporte une paroi extérieure 142 qui est ici de forme cylindrique, et se termine par un fond 110 sensiblement conique, la partie supérieure de ladite enceinte étant fermée par une paroi supérieure 121. Le lit 113 repose sur une grille de support 111, dont les orifices sont de préférence équipés de tuyères à chapeau 112 de conception traditionnelle, ces tuyères, soudées à la grille, permettant d'éviter tout siphonnage du lit vers le bas à l'arrêt du processus (à l'arrêt, il peut ainsi se former de petits talus au voisinage des tuyères, sans qu'il y ait siphonnage vers le bas d'une partie du lit par les orifices, comme ce serait le cas avec une simple grille plate, ce qui serait particulièrement désavantageux à la reprise du processus en raison des risques d'obturation partielle de la grille, avec par suite une altération du champ de vitesses de l'air de fluidisation). L'air de fluidisation est généré par un ventilateur 115, raccordé au fond 110 de l'enceinte 101 par une conduite associée 114.

Le lit dense 113 est de préférence un lit de sable, dont la hauteur efficace h est choisie en fonction du diamètre moyen des particules de silice à traiter. Cette hauteur efficace h correspond à la hauteur du lit dense 113 entre le niveau d'introduction de la poudre à traiter, c'est-à-dire ici le niveau où débouche une vis de transport 108, et la surface du lit 116. Une chambre de désengagement 117, ou ciel de lit, est ainsi délimitée entre la surface du lit 116 et la paroi supérieure 121 de l'enceinte fermée 101. On pourra par exemple choisir un lit dense 113 constitué par des particules de sable dont la granulométrie moyenne est de l'ordre de 400 µm. Dans ce cas, la hauteur efficace h du lit dense 113 sera de l'ordre de 1,5 mètre lorsque ledit lit est fluidisé (ceci correspond à une hauteur d'environ 1,3 mètre lorsque l'installation est au repos, et que le lit dense n'est pas fluidisé). Dans ce cas, on pourra choisir un débit d'air de fluidisation, donné par le ventilateur 115, qui sera de l'ordre de 140 Nm³/h pour un diamètre de l'enceinte voisin de un mètre.

Le chauffage du lit dense 113 peut être organisé par l'extérieur, comme cela est illustré ici, à l'aide de moyens de chauffage électrique, qui sont de préférence réalisés sous la forme de moufles chauffants 118, 119 disposés contre la paroi extérieure 142 de l'enceinte fermée 101. Chacun de ces moufles chauffants comporte alors une résistance de chauffage 143, et l'on peut utiliser deux ou trois ou encore quatre coquilles constituant un véritable manchon chauffant qui entoure la paroi extérieure de l'enceinte fermée (en variante, ce manchon chauffant pourrait être réalisé par des anneaux enfilés successivement sur l'enceinte fermée). Lorsqu'il s'agit d'une installation de grande dimension, par exemple destinée à traiter plus de trois tonnes de poudre par heure, on pourra alors remplacer les moufles chauffants illustrés ici par un thermoplongeur immergé dans le lit dense, de préférence coaxialement à celui-ci, le chauffage du lit se faisant alors par l'intérieur et sensiblement à partir de l'axe de l'enceinte fermée. Les moyens de chauffage 120, constitués ici par les moufles chauffants 118 et 119, s'étendent de préférence sur une hauteur au moins sensiblement égale à la hauteur efficace h prédéterminée du lit dense fluidisé 113. Ainsi, le lit dense 113 est chauffé sur la totalité de sa hauteur efficace h, de façon que tout l'air de fluidisation soit de l'air comburant pour oxyder le carbone présent dans les fumées de silice à traiter, la température dudit lit étant cependant choisie inférieure à la température de frittage de la poudre à traiter. La température du lit dense doit être choisie aussi élevée que possible afin qu'un temps de contact minimum soit suffisant pour assurer le blanchiment de la poudre à traiter, avec par suite une taille aussi faible que possible pour l'enceinte fermée. Cette température doit en tout état de cause être inférieure à la température de frittage de la poudre afin d'éviter un phénomère de collage qui induirait une réaggrégation des particules, et bouleverserait alors l'hydrodynamique du système. On choisira de préférence une température du lit dense 113 réglée entre environ 550°C et 850°C pour oxyder complètement le carbone sans modifier l'état cristallographique des fumées de silice. Il convient de noter que le lit dense fluidisé 113 constitue ce qu'on appelle un lit "infiniment mélangé", c'est-à-dire qu'il n'y a pratiquement aucune différence de température entre les différents points du lit dense. De ce fait, lorsque la poudre à traiter traverse le lit dense 113 sur toute la hauteur efficace h de celui-ci, on est assuré d'un traitement homogène de cette poudre, ce qui garantit l'obtention de la couleur blanche désirée lorsque les particules s'échappent dudit lit dense. Il s'agit donc d'un lit à phase dense (dénommé "bubble bed" par les anglo-saxons), dans la mesure où les particules de sable restent confinées géométriquement dans un volume bien défini, sans en sortir. A titre indicatif, il est possible de contrôler le système de telle façon que les écarts de température entre le bas du lit, au voisinage de la grille de support, et une zone moyenne dudit lit, n'excèdent pas 20°C. On peut donc considérer que l'ensemble du lit dense 113 est à une température globalement homogène, ce qui est naturellement favorable pour les échanges thermiques. On constate finalement que l'enceinte fermée 101 constitue ici un véritable réacteur isotherme.

Lorsque l'enceinte fermée 101 contient un lit dense 113 de sable dont la hauteur efficace h est de l'ordre de 1,5 mètre (hauteur du lit fluidisé), on pourra choisir une hauteur de quelques mètres, par exemple au moins trois mètres pour la chambre de désengagement 117. Le diamètre de l'enceinte fermée 101 est moins critique, mais doit cependant être supérieur à une valeur minimale pour éviter tout risque de formation d'une succession de pistons de solide/gaz, ce qui nuirait à un bon échange thermique : avec une hauteur de 1,5 mètre pour le lit dense fluidisé 113, on pourra choisir pour l'enceinte fermée un diamètre de l'ordre d'un mètre.

Conformément à un aspect essentiel du procédé de traitement selon l'invention, la poudre noire à traiter 105 est introduite en partie basse du lit dense 113 fluidisé et chauffé, ledit lit présentant une hauteur efficace h prédéterminée permettant d'oxyder en gaz carbonique le carbone des particules de silice lorsque celles-ci traversent le lit dense fluidisé en étant entraînées par l'air de fluidisation, la poudre alors blanchie 131 étant récupérée par filtration des gaz issus dudit lit dense fluidisé.

On trouve donc des moyens d'introduction 102 et de stockage 103 de la poudre à traiter, moyens qui sont disposés au voisinage de l'enceinte fermée 101 contenant le lit dense fluidisé 113. La poudre noire à traiter, que constituent les fumées de silice 105, est stockée dans une trémie 104 à la base de laquelle est prévue un sas rotatif doseur (non représenté ici) alimentant, par une conduite 106, un canal d'introduction 107 qui débouche latéralement en bas de l'enceinte fermée 101, c'est-à-dire en partie basse du lit dense fluidisé 113. On pourra utiliser, comme cela est représenté ici, une vis sans fin de transport 108, entraînée par un moteur associé 109, cette vis de transport étant sensiblement horizontale, et débouchant à l'intérieur de l'enceinte fermée 101, de préférence en pénétrant légèrement à l'intérieur de ladite enceinte (par exemple en débouchant de 5 cm dans celle-ci). Il va de soi que la vis de transport de type Archimède 108 pourra être remplacée par un organe de transport pneumatique en phase dense adapté à la contrepression du lit.

La poudre noire à traiter 105 est ainsi introduite en partie basse du lit dense fluidisé 113, et cette poudre est "retenue" temporairement par la couche de sable fluidisée, qui assure un temps de mise en contact convenable avec le lit dense qui est fluidisé et chauffé à la température désirée, ce qui permet au carbone de s'oxyder en gaz carbonique. Lorsque la poudre atteint la surface du lit 116, elle n'a plus de carbone, et s'envole avec les gaz issus du lit fluidisé 113, et il suffit alors d'organiser un circuit de séparation convenable pour récupérer la poudre blanchie 131.

Il est intéressant de noter que, sans la présence d'un lit dense fluidisé de sable, la poudre noire à traiter s'envolerait avec la vitesse ascensionnelle de l'air de fluidisation. Le fait que cette poudre soit "retenue" par le lit dense fluidisé constitue une approche originale allant à l'encontre d'un préjugé pour l'homme de l'art, dans la mesure où une couche fluidisée de poudre serait impossible à traiter telle quelle.

On va maintenant décrire le circuit de séparation permettant de récupérer aisément la poudre blanchie 131, ledit circuit comportant des moyens de filtration 126 des gaz issus du lit dense fluidisé 113, et des moyens de récupération 130 de la poudre blanchie.

La sortie de l'enceinte fermée 101 est constituée par une conduite 122 menant à l'entrée d'un séparateur cyclone 123, dont la sortie 124 est reliée à une conduite 125 menant à l'entrée des moyens de filtration 126. Ainsi, l'entrée du séparateur cyclone 123 est directement reliée à la chambre de désengagement 117 de l'enceinte fermée 101, de sorte que les gaz issus du lit dense fluidisé 113 passent par ce séparateur cyclone 123, ce qui permet de ramener les éventuelles particules de sable vers le lit dense 113, grâce à une communication 141 prévue entre l'extrémité basse du cyclone et la chambre de désengagement 117, la filtration qui permet de récupérer la poudre blanchie étant alors effectuée en aval de ce séparateur cyclone. On choisira de préférence un séparateur cyclone présentant un diamètre de coupure de l'ordre de 30 μm, ce qui permet d'éviter que la silice blanchie ne soit polluée par des particules de sable.

Les moyens de filtration 126 sont de préférence constitués par un filtre à manches 127, dont la partie inférieure aboutit à un sas rotatif coupe-pression 128 sous lequel est disposé un bac 129 constituant les moyens de récupération, et dont la partie supérieure est reliée par une conduite associée 136 à un ventilateur d'exhaure 137 évacuant les gaz vers une cheminée 138. Le sas rotatif 128 comporte un orifice de sortie 135 par lequel peut s'écouler la poudre blanchie 131 ainsi récupérée dans le bac 129. Le bac 129 sera de préférence un sac souple en textile, fermé inférieurement par une attache 145 (simple ou multiple selon que les parois du sac sont à simple couche ou multicouches), et présentant en partie haute un voile de fermeture 134 et des lanières de préhension 133. Un tel sac est ici posé sur un support 132, avant d'être saisi par les lanières 133. Les sacs de ce type dénommés "big bag" par les anglo-saxons, sont couramment utilisés dans le domaine des conteneurs maritimes : ils offrent un volume utile d'environ 1 m³, et présentent en général une enveloppe et une jupe constituant une double paroi, avec quatre lanières de préhension, et une double attache inférieure qui est simplement ouverte pour vider le sac.

On utilise de préférence, pour les moyens de filtration 126, un filtre à manches 127 de conception classique, comportant des chaussettes en textile (non visibles ici) dont le diamètre de coupure est de préférence inférieur ou égal à 1 μm. Ce type de filtre est bien connu, et il comporte son propre système de décolmatage mécanique, par voie de secousses, système qui est également équipé d'un collecteur de récupération en partie basse du filtre. Le sas rotatif 128 permet de récupérer la poudre blanchie 131 sans rupture de la pression régnant dans le filtre à manches 127 (on dénomme de ce fait ce type de sas "sas coupe-pression"). En utilisant un filtre à manches dont le dimensionnement est comparable à celui donné plus haut pour l'enceinte fermée 101, on pourra utiliser un débit de gaz compris entre 1 000 et 2 000 Nm³/h, ce qui permet, avec les dimensionnements donnés plus haut pour l'enceinte fermée 101 et pour la hauteur du lit dense fluidisé contenu dans ladite enceinte, de traiter au moins 600 kg de poudre par heure.

Toutefois, lorsque l'on utilise un filtre à manches en textile, il est en général nécessaire de faire en sorte que la température des gaz filtrés ne dépasse pas environ 200°C (certains matériaux textiles récents peuvent cependant résister à des températures atteignant 240°C). A cet effet, la conduite 125 reliant la sortie du séparateur cyclone 123 à l'entrée du filtre à manches 127 comporte une dérivation ouverte 139 permettant d'admettre dans ladite conduite de l'air froid de dilution. La température des gaz chauds sortant du séparateur cyclone 123 peut alors être aisément ramenée à environ 150°C lorsque ces gaz arrivent au filtre à manches 127. Les gaz sortant du séparateur du cyclone 123 subissent ainsi une dilution efficace par cet air frais pour abaisser leur température avant que ces gaz soient filtrés pour la récupération de la poudre blanchie 131. De préférence, la dérivation 139 comporte un organe de réglage 140, tel qu'une clé, pour régler la quantité d'air de dilution arrivant dans cette conduite 125.

Une installation du type qui vient d'être décrit ne requiert qu'une puissance installée de l'ordre de 150 kW. Si l'on souhaite réaliser des installations plus puissantes, afin notamment de dépasser une masse de traitement supérieure à 3 tonnes par heure, la puissance requise dépassera alors 500 kW et l'on utilisera une enceinte fermée de plus gros diamètre, avec des moyens d'introduction organisés en plusieurs points répartis sur la périphérie de l'enceinte fermée, pour que les particules soient réparties uniformément dans la section du lit dense fluidisé. Dans le cas de gros diamètres, il sera en général également nécessaire de prévoir un thermoplongeur pour assurer le chauffage uniforme du lit dense fluidisé, ainsi que cela a été dit plus haut.

Une puissance de l'ordre de 150 kW sera la plupart du temps suffisante pour obtenir aisément une température réglable entre 550 et 750°C pour le lit dense fluidisé, grâce aux mouffles chauffants électriques disposés autour de la paroi du lit et rayonnant sur l'enceinte fermée. Les mouffles chauffants électriques pourront naturellement être réalisés sous la forme d'anneaux empilés, en variante d'une réalisation en portions de coquille cylindrique conformément à ce qui a été décrit plus haut. La gamme de températures précitée sera en général suffisante pour oxyder complètement le carbone sans modifier l'état cristallographique des fumées de silice. Il sera toutefois intéressant, dans certaines applications, de pouvoir traiter les fumées de silice à une température atteignant 850°C, ce qui permet en particulier de proposer aux élaborateurs de cosmétiques un produit légèrement différent du produit de base par sa proportion de cristoballite.

L'installation qui vient d'être décrite peut être aisément pilotée, en utilisant une régulation basée sur l'écart mesuré au moyen d'une thermosonde (non représentée sur la figure) entre la température dans une couche moyenne du lit dense fluidisé et la température en bas de ce lit (par exemple à quelques centimètres de la grille de support). Lorsque l'écart mesuré reste inférieur par exemple à un seuil de 20°C, ceci signifie que l'état fluidisé est atteint avec de bonnes conditions. Dès que l'écart dépasse ce seuil de 20°C, cette information est transmise à un automate de conduite, de façon à agir en conséquence sur la commande du ventilateur produisant l'air de fluidisation, pour obtenir une augmentation de débit. Un algorithme convenable de régulation prévoira alors le déclenchement d'une alerte, ou encore l'arrêt de l'installation si l'état fluidisé n'est toujours pas atteint au bout d'un temps donné.

Une telle régulation thermique permet de s'assurer que le lit dense fluidisé reste en permanence à une température homogène dans toute sa masse, ce qui garantit un choc thermique uniforme et important pour le produit à traiter qui est ainsi porté très brutalement à une température élevée (avec un temps de l'ordre de la seconde), un tel choc thermique étant naturellement très favorable pour oxyder complètement les particules de carbone.

L'installation qui vient d'être décrite, avec les dimensionnements qui ont été donnés plus haut, ne nécessite qu'une surface au sol d'environ 30 m², et une hauteur d'environ 10 m. La puissance électrique requise reste également modique, car une installation de ce type ne demande qu'un branchement électrique triphasé de 380 Volts d'une puissance de 200 kVA. La conduite par automate est avantageuse dans la mesure où l'installation ne demande pas de personnel d'exploitation. Le personnel de maintenance est alors simplement averti par l'équipement de télé-alarme de l'installation de tout aléa de fonctionnement. Pour le site industriel, on peut choisir de connecter l'installation au reste de l'usine par des dispositifs automatisés d'alimentation de fumées de silice dans la trémie et de collecte du produit fini.

Le procédé et le dispositif qui viennent d'être décrits ne constituent naturellement qu'un exemple non limitatif, et pourront subir des modifications par utilisation de phase ou moyens équivalents. En particulier, il sera possible de supprimer le séparateur cyclone en prévoyant un ciel de lit très évasé, avec une récupération à hauteur élevée (de l'ordre de 10 mètres environ) : toutefois, l'installation sera naturellement plus encombrante en hauteur.

Le procédé et le dispositif de l'invention trouvent une application particulièrement avantageuse dans le domaine des cosmétiques. En effet, la poudre récupérée à l'issue du traitement qu'elle a subi présente une couleur blanche satisfaisante, tout en conservant une granulométrie très fine propice à l'effet d'onctuosité recherché pour les produits cosmétiques, en particulier les fonds de teint et les "poudres de riz". Par ailleurs, le mode de traitement conforme à l'invention permet de préserver la sphéricité des particules de silice blanchies, ce qui est hautement favorable à l'obtention d'un degré élevé d'onctuosité.

L'invention n'est pas limitée aux modes de réalisation qui viennent d'être décrits mais englobe au contraire toute variante reprenant, avec des moyens équivalents, les caractéristiques essentielles énoncées plus haut.

**Revendications**

1/ Procédé de traitement de fumées de silice en vue de leur blanchiment, caractérisé par le fait que la poudre noire à traiter (105) est introduite en partie basse d'un lit dense (113) fluidisé et chauffé, ledit lit présentant une hauteur efficace (h) prédéterminée entre le niveau d'introduction et la surface du lit permettant d'oxyder en gaz carbonique le carbone des particules de silice lorsque celles-ci traversent le lit dense fluidisé (113) en étant entraînées par l'air de fluidisation, la poudre alors blanchie (131) étant récupérée par filtration des gaz issus dudit lit dense fluidisé.

2/ Procédé de traitement selon la revendication 1, caractérisé par le fait que le lit dense (113) est un lit de sable, dont la hauteur efficace (h) est choisie en fonction du diamètre moyen des particules de silice à traiter.

3/ Procédé de traitement selon la revendication 2, caractérisé par le fait que le diamètre moyen des particules de sable constituant le lit dense (113) est de l'ordre de 400 µm.

4/ Procédé de traitement selon la revendication 3, caractérisé par le fait que la hauteur efficace (h) du lit dense (113) est de l'ordre de 1,5 mètre lorsque ledit lit est fluidisé.

5/ Procédé de traitement selon l'une des revendications 1 à 4, caractérisé par le fait que le lit dense (113) est chauffé par sa surface extérieure, par chauffage électrique.

6/ Procédé de traitement selon la revendication 5, caractérisé par le fait que le lit dense (113) est chauffé sur la totalité de sa hauteur efficace (h), de façon que tout l'air de fluidisation soit de l'air comburant pour oxyder le carbone présent dans les fumées de silice, la température dudit lit restant cependant inférieure à la température de frittage de la poudre à traiter (105).

7/ Procédé de traitement selon la revendication 5 ou 6, caractérisé par le fait que la température du lit dense (113) peut être réglée entre environ 550°C et 850°C pour oxyder complètement le carbone sans modifier l'état cristallographique des fumées de silice.

8/ Procédé de traitement selon l'une des revendications 2 à 7, caractérisé par le fait que les gaz issus du lit dense fluidisé (113) passent par un séparateur cyclone (123), pour ramener les éventuelles particules de sable vers le lit dense (113), la filtration qui permet de récupérer la poudre blanchie étant effectuée en aval dudit séparateur cyclone.

9/ Procédé de traitement selon la revendication 8, caractérisé par le fait que la séparation opérée par le séparateur cyclone (123) est obtenue par le choix d'un diamètre de coupure de l'ordre de 30 µm pour ledit séparateur cyclone.

10/ Procédé de traitement selon la revendication 8 ou 9, caractérisé par le fait que les gaz sortant du séparateur cyclone (123) subissent une dilution avec de l'air frais pour abaisser leur température avant que ces gaz soient filtrés pour la récupération de la poudre blanchie (131).

11/ Procédé de traitement selon la revendication 10, caractérisé par le fait que la température des gaz est ramenée à moins de 150°C avant leur filtration.

12/ Dispositif de mise en oeuvre du procédé de traitement selon l'une des revendications 1 à 11, caractérisé par le fait qu'il comporte une enceinte fermée (101) contenant un lit dense (113) fluidisé par des moyens d'amenée d'air (111, 112, 114, 115) et maintenu à une température déterminée par des moyens de chauffage (120) associés, des moyens d'introduction (102) de la poudre noire à traiter (105) en partie basse du lit dense fluidisé (113), ledit lit présentant une hauteur efficace (h) prédéterminée entre le niveau d'introduction et la surface du lit, et ladite enceinte se prolongeant supérieurement, au-dessus de la surface du lit dense fluidisé (113), par une chambre de désengagement (117) reliée à un circuit de séparation pour récupérer la poudre blanchie (131), ledit circuit de séparation comportant des moyens de filtration (126) des gaz issus du lit dense fluidisé (113) et des moyens de récupération (130) de la poudre blanchie.

**13/** Dispositif selon la revendication 12, caractérisé par le fait que l'enceinte fermée (101) est métallique, et sa paroi extérieure (142) est chauffée par des moufles chauffants électriques (118, 119).

**14/** Dispositif selon la revendication 13, caractérisé par le fait que les moufles chauffants électriques (118, 119) s'étendent sur une hauteur au moins sensiblement égale à la hauteur efficace (h) prédéterminée du lit dense fluidisé (113).

**15/** Dispositif selon l'une des revendications 12 à 14, caractérisé par le fait que l'enceinte fermée (101) contient un lit dense (113) de sable dont la hauteur efficace (h) est de l'ordre de 1,5 mètre lorsque ledit lit est fluidisé, et présente une chambre de désengagement (117) dont la hauteur est de plusieurs mètres, en particulier au moins trois mètres.

**16/** Dispositif selon la revendication 15, caractérisé par le fait que l'enceinte fermée (101) est de l'ordre d'un mètre.

**17/** Dispositif selon l'une des revendication 12 à 16, caractérisé par le fait que les moyens d'amenée d'air (111, 112, 114, 115) comportent une conduite unique d'amenée (114) raccordée à l'enceinte fermée (101) par le fond (110) de celle-ci.

**18/** Dispositif selon l'une des revendications 12 à 17, caractérisé par le fait que les moyens d'introduction (102) de la poudre noire à traiter (105) comportent une trémie (104) et une vis de transport (108) sensiblement horizontale débouchant à l'intérieur de l'enceinte fermée (101).

**19/** Dispositif selon l'une des revendications 12 à 18, caractérisé par le fait que le circuit de séparation comporte un séparateur cyclone (123) dont l'entrée est directement reliée à la chambre de désengagement (117) de l'enceinte fermée (101), le diamètre de coupure dudit séparateur cyclone étant de préférence choisi voisin de 30 µm.

**20/** Dispositif selon l'une des revendications 12 à 19, caractérisé par le fait que les moyens de filtration (126) sont essentiellement constitués par un filtre à manches (127), dont la partie inférieure aboutit à un sas rotatif (128) sous lequel est disposé un bac (129) constituant les moyens de récupération, et dont la partie supérieure est reliée à un ventilateur d'exhaure (137) évacuant les gaz vers une cheminée (138).

**21/** Dispositif selon la revendication 20, caractérisé par le fait que le filtre à manches (127) est en textile, et présente un diamètre de coupure au plus égal à 1 µm.

**22/** Dispositif selon la revendication 19 et la revendication 20 ou 21, caractérisé par le fait que la conduite (125) reliant la sortie du séparateur cyclone (123) à l'entrée du filtre à manches (127) comporte une dérivation ouverte (139) permettant d'admettre dans ladite conduite de l'air froid de dilution.

**23/** Dispositif selon la revendication 22, caractérisé par le fait que la dérivation (139) comporte un organe de réglage (140) tel qu'une clé, pour régler la quantité d'air de dilution arrivant dans ladite conduite (125).

**EP 0 491 611 A1**

Office européen
des brevets

**RAPPORT DE RECHERCHE EUROPEENNE**

Numero de la demande

EP    91 40 3404

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 86, no. 24, 13 juin 1977, page 138, abrégé no. 173719v, Columbus, Ohio, US; & JP-A-77 13 497 (CHISSO CORP.) 01-02-1977 --- | | C 01 B   33/12 B 01 J    8/24 |
| A | EP-A-0 147 617   (BERGWERKSVERBAND GmbH) ----- | | |

DOMAINES TECHNIQUES RECHERCHES (Int. Cl.5)

C 01 B
B 01 J

Le présent rapport a été établi pour toutes les revendications

| Lieu de la recherche | Date d'achèvement de la recherche | Examinateur |
|---|---|---|
| LA HAYE | 25-03-1992 | BREBION J.CH. |

EPO FORM 1503 03.82 (P0402)